Europäisches Patentamt

(19) European Patent Office

Office européen des brevets

(11) Numéro de publication : **0 350 374 B1**

(12) **FASCICULE DE BREVET EUROPEEN**

(45) Date de publication du fascicule du brevet :
28.08.91 Bulletin 91/35

(51) Int. Cl.⁵ : **C12N 11/04**

(21) Numéro de dépôt : **89401867.0**

(22) Date de dépôt : **29.06.89**

(54) **Procédé de préparation de micro-organismes inclus dans des gels sensiblement déshydratés, gels obtenus et leur utilisation pour la préparation de boissons fermentées.**

(30) Priorité : 07.07.88 FR 8809249

(43) Date de publication de la demande :
10.01.90 Bulletin 90/02

(45) Mention de la délivrance du brevet :
28.08.91 Bulletin 91/35

(84) Etats contractants désignés :
AT BE CH DE ES GB GR IT LI LU NL SE

(56) Documents cités :
EP-A- 0 065 376
EP-A- 0 083 267
FR-A- 2 586 256
FR-A- 2 600 673

(72) Inventeur : **Divies, Charles**
**90, rue des Péjoces**
**F-21000 Dijon (FR)**
Inventeur : **Lenzi, Pascal**
**27, Boulevard de l'Université**
**F-21000 Dijon (FR)**
Inventeur : **Beaujeu, Jacques**
**11, rue Lenez Magenta-Nouméa**
**Nouvelle Calédonie (FR)**
Inventeur : **Herault, Frédéric**
**16, rue de la Blanchetière**
**F-44240 La Chapelle sur Erdre (FR)**

(74) Mandataire : **Portal, Gérard et al**
**Cabinet Beau de Loménie 55, rue**
**d'Amsterdam**
**F-75008 Paris (FR)**

(73) Titulaire : **CHAMPAGNE MOET & CHANDON**
**20 avenue de Champagne**
**F-51200 Epernay (FR)**

EP 0 350 374 B1

## Description

La présente invention concerne essentiellement un procédé de préparation de micro-organismes inclus dans des gels sensiblement déshydratés, gels obtenus et leur utilisation pour la préparation de boissons fermentées.

La présente invention a été réalisée dans le cadre du laboratoire de microbiologie de l'Ecole Nationale Supérieure de Biologie Appliquée à la Nutrition et à l'Alimentation (ENSBANA).

Dans l'état de la technique antérieure, il est connu que les micro-organismes immobilisés peuvent servir à l'élaboration de boissons fermentées telles que le vin et la bière (FR-A-2320349 et FR-A-2359202). On a également proposé leur utilisation pour la champagnisation classique (FR-A-2432045) ainsi que pour la fabrication de boissons pétillantes à degré d'alcool variable (FR-A-2601687). Ces publications, ainsi que d'autres (notamment JP-A-57-150385 ou EP-A-173915) ont souligné les performances des réacteurs à cellules immobilisées.

Ces techniques ont rendu possible la réalisation des fermentations avec des mélanges de micro-organismes de différentes catégories (mélange de bactéries lactiques et mélange de levures).

Cependant, la mise en oeuvre du procédé au niveau industriel s'est heurtée à la difficulté de disposer de particules pouvant se conserver pendant une durée importante.

Les micro-organismes sous forme incluse peuvent être utilisés sans baisse notable d'activité sur de longues périodes lorsqu'on respecte la nutrition des micro-organismes concernés.

On a pu constater une toxicité des produits de fermentation qui entraîne un vieillissement partiel des cellules (article de DIVIES et al. dans Annales de microbiologie, 1977, pages 349-358).

Il a donc été préféré dans ce cas précis d'utiliser une batterie de réacteur à durée de vie déterminée et de procéder au renouvellement partiel de manière programmée des particules microbiennes.

Des problèmes de résistance mécanique du gel ont pu également surgir et sont décrits dans JP-A-57-150 385.

Il apparaît donc crucial au niveau industriel de planifier l'élaboration des particules d'inclusion des micro-organismes et d'en centraliser la production. Il est également nécessaire d'obtenir une inclusion des micro-organismes dans les gels qui leur assure une excellente viabilité au cours du temps.

Or, il est apparu que les micro-organismes inclus dans des gels étaient très sensibles et supportaient difficilement une conservation pendant une période de temps prolongée.

Pour remédier à cet inconvénient de la conservation, on a déjà proposé des procédés de conservation incluant un séchage.

Les préparations commerciales de micro-organismes inclus dans des gels séchés doivent être naturellement aptes à une réhydratation et conserver une excellente viabilité après réhydration.

Certaines solutions ont été proposées permettant une conservation sur des périodes pouvant atteindre 6 mois à 1 an dans un emballage protecteur à température relativement basse de 4 à 10°C (voir BECKER et RAPOPORT dans Advances in Biochemical Engineering and Biotechnology, Volume 35, 1987, pages 128 à 171).

Le document TATE EP-A-0065376 décrit un procédé de préparation d'enzymes immobilisés dans un gel qui est ensuite éventuellement séché, et après séchage, mis en contact avec du glycérol (page 10, 2ème paragraphe et revendication 3). Une mise en contact après séchage ne permet pas de conserver la structure du gel, ce qui rend difficile sa réhydratation, problème qui est résolu par la présente invention qui sera décrite plus loin.

Le document FR-A-2519022 décrit un procédé de préparation d'inoculums à longue viabilité et ayant une résistance à la température améliorée comprenant un séchage des micro-organismes selon divers procédés de séchage. Ce document décrit également dans sa partie introductive de nombreux documents de la technique antérieure relatifs à un séchage de gels incluant des micro-organismes.

Selon ce document FR-A-2519022, on réalise au départ une culture de micro-organismes dans un milieu de culture classique pendant plusieurs jours.

A ce milieu de culture, on peut ajouter un polymère gélifiable pouvant être un polysaccharide, du xanthane ou un alginate.

Après gélification permettant de réaliser l'inclusion d'un micro-organisme dans le milieu de culture, on procède à un séchage jusqu'à obtention d'une activité de l'eau dans l'inoculum en dessous de sa valeur critique à une valeur inférieure à 0,5, cette valeur étant maintenue lors de la conservation (voir revendication 1 notamment). De préférence, l'activité de l'eau dans l'inoculum est maintenue en-dessous de 0,3 et encore de préférence en-dessous de 0,1 (revendication 2).

Il est à observer que selon ce document on ne s'attache pas au problème particulier de la réhydratation du gel sensiblement déshydraté ou séché, de manière à obtenir des gels réhydratés ayant une structure sen-

EP 0 350 374 B1

siblement identique à celle qu'ils possédaient avant leur déshydratation.

Or, l'expérience a montré que les particules déshydratées ou séchées obtenues par le procédé décrit dans ce document se réhydratent très difficilement. Dans le meilleur des cas, en présence de milieu de culture, la réhydratation reste limitée avec 20% d'humidité, les particules restant toujours très petites, flétries, non calibrées et très dures.

Si l'on réalise une supplémentation en substances hydrophiles telles que les carraghénanes ou la farine de caroube, on provoque une fragilisation du gel sans amélioration de la réhydratation du polymère gélifié, et ceci est particulièrement vrai dans le cas de l'emploi d'un alginate. Dans le meilleur des cas, on peut obtenir des viabilités de quelques pour-cent incompatibles avec une utilisation industrielle.

Il apparaît donc nécessaire de disposer de gels facilement réhydratables comprenant un maximum de micro-organismes viables après réhydratation, et ceci même après une longue période de stockage.

La présente invention a donc pour but de résoudre le nouveau problème technique consistant en la fourniture d'une solution permettant de disposer de gels séchés ou essentiellement déshydratés, facilement réhydratables, comprenant un maximum de micro-organismes viables après réhydratation, et ce même après une période prolongée de conservation.

La présente invention a aussi pour but de résoudre le nouveau problème technique consistant en la fourniture d'une solution permettant de disposer de gel, comprenant un maximum de micro-organismes viables après réhydratation, conservant une structure essentiellement inchangée du gel après réhydratation, assurant une bonne stabilité de ce gel compatible avec une utilisation industrielle du gel réhydraté.

Ces problèmes techniques sont résolus simultanément pour la première fois par la présente invention d'une manière extrêmement simple, utilisable à l'échelle industrielle.

Ainsi, selon un premier aspect, la présente invention fournit un procédé de préparation de micro-organismes inclus dans des gels au moins partiellement déshydratés présentant une viabilité améliorée après réhydratation, comprenant :

a) l'inclusion de micro-organismes dans une solution de polymère susceptible de se transformer en gel ;

b) la gélification de cette solution contenant les micro-organismes de manière à former un gel incluant les micro-organismes ; et

c) le séchage de ce gel incluant les micro-organismes, pour obtenir un gel au moins partiellement déshydraté, caractérisé en ce qu'on prépare des gels incluant les micro-organismes ayant une forte concentration en substances hydrophiles que l'on soumet ensuite à ladite étape de séchage.

On entend par "forte concentration", une concentration en substances hydrophiles nettement supérieure à la concentration d'utilisation habituelle de cette substance comme agent de protection de séchage. De préférence, cette concentration en substances hydrophiles est au moins deux fois la concentration habituelle, encore mieux au moins cinq fois la concentration habituelle.

Selon une autre caractéristique avantageuse du procédé selon l'invention, la substance hydrophile précitée est d'un faible poids moléculaire. Cette substance hydrophile est de préférence choisie parmi les polyols tels que le sorbitol, l'inositol, le glycérol ; les sucres tels que le saccharose, le glucose, le fructose. Le saccharose constitue le sucre particulièrement préféré car il permet d'obtenir des résultats particulièrement inattendus.

Selon un mode de réalisation particulièrement avantageux du procédé selon l'invention, la concentration en saccharose est au moins égale à 500 g/l, de préférence environ 1000 g/l de solution de polymère susceptible de se transformer en gel.

Selon une autre caractéristique avantageuse du procédé selon l'invention, on réalise une culture des cellules de micro-organismes jusqu'à obtention de la phase stationnaire, ce qui correspond en particulier pour les levures à un faible taux de bourgeonnement ou d'initiation de division. De préférence, ce faible taux de bourgeonnement est inférieur à 5%. Ceci permet d'augmenter de manière inattendue la viabilité des cellules de micro-organismes, et en particulier dans le cas des levures.

Selon une autre caractéristique avantageuse du procédé selon l'invention, on prépare un gel ayant une structure double couche comprenant une couche interne ou noyau de gel contenant les cellules de micro-organismes et une couche externe ou enveloppe de gel sensiblement exempt de micro-organismes. Ce gel peut présenter la forme soit de billes, soit de fibres, comme cela est bien connu dans les techniques de gélification.

Pour réaliser un gel ayant une structure double couche, on peut utiliser les procédés antérieurement connus, tels que décrits par exemple dans le document JP-A-57-150385 en utilisant de préférence le procédé décrit dans ce document qui consiste à former la couche externe ou enveloppe avec une solution gélifiable. On peut également utiliser la technique décrite dans le document GB-A-1158662 au US-A-4386895 ou US-A-3396116, ou encore EP-A-0140336 ou US-A-3015128 ou US-A-3310612 ou encore les techniques de préparation d'inclusion décrites dans un article de P.G. Krouvel dans Biotechnology and bioengineering (1980), volume 22, page 681 ou le document Microcapsules Processing and Technology (Asaji Kondo) 1979, pages 62 à 66.

3

EP 0 350 374 B1

Selon une caractéristique particulièrement avantageuse du procédé selon l'invention, l'épaisseur de la couche externe ou enveloppe, dans le cas de billes ayant un diamètre d'environ 4 mm, est inférieure à 0,8 mm.

Selon une autre caractéristique avantageuse du procédé selon l'invention, on obtient la forte concentration en substances hydrophiles précitée dans le gel par trempage du gel dans une solution de substances hydrophiles ayant ladite forte concentration, jusqu'à l'équilibre, puis en soumettant le gel à l'étape de séchage précitée.

Selon une autre caractéristique avantageuse du procédé selon l'invention, on réalise un séchage du gel incluant les microorganismes, ayant la forte concentration précitée en substances hydrophiles, jusqu'à obtention d'une activité de l'eau inférieure à environ 0,5.

Selon une autre caractéristique avantageuse du procédé selon l'invention, on réalise le séchage précité à une température voisine de 40°C et de préférence on augmente la température jusqu'à atteindre environ 50°C en fin de séchage.

Selon une autre caractéristique avantageuse du procédé selon l'invention, on réalise le séchage précité du gel incluant les micro-organismes, comprenant une forte concentration en substances hydrophiles, dans un lit fluidisé.

Selon une autre caractéristique du procédé selon l'invention, on réalise un séchage du gel contenant les micro-organismes, comprenant la forte concentration précitée en substances hydrophiles par une technique de lyophilisation sous vide, de préférence la température de lyophilisation est de l'ordre de −80°C ± 10°C environ.

Selon une autre caractéristique avantageuse du procédé selon l'invention, on conserve le gel contenant les micro-organismes, comprenant la forte concentration en substances hydrophiles précitée, au moins partiellement déshydratées, dans un emballage étanche à la vapeur d'eau, qui est de préférence maintenu à une température relativement basse, de préférence d'environ 4°C.

Selon une caractéristique préférée, cette conservation a lieu dans une atmosphère contrôlée très appauvrie en oxygène et enrichie en $CO_2$.

Selon une autre caractéristique avantageuse du procédé selon l'invention, les micro-organismes sont des levures, en particulier du genre Saccharomyces et Schizosaccharomyces.

Selon un deuxième aspect, la présente invention concerne aussi l'utilisation des gels incluant des micro-organismes, au moins partiellement déshydratés précités, pour la préparation de boissons fermentées ou refermentées, ainsi que pour la préparation d'alcool éthylique.

La présente invention concerne également les gels incluant les micro-organismes, ayant la forte concentration précitée en substances hydrophiles, à titre de produit nouveau, éventuellement à un état réhydraté.

L'invention permet d'aboutir aux résultats techniques inattendus, non évidents précédemment mentionnés, en résolvant les nouveaux problèmes techniques précédemment énoncés en découvrant de manière inattendue que l'on pouvait conserver à la fois la structure des gels et la viabilité des micro-organismes si l'on utilisait une forte concentration en substances hydrophiles. Comme il a été mentionné précédemment, ces substances hydrophiles sont de préférence choisies parmi les polyols tels que le sorbitol, l'inositol, le glycérol ; les sucres, en particulier le saccharose, le glucose, le fructose ; le sucre davantage préféré actuellement est le saccharose. La forte concentration en saccharose est de préférence d'au moins 500 g/l, encore de préférence environ 1000 g/l de solution de polymère susceptible de se transformer en gel.

On obtient ainsi des gels au moins partiellement déshydratés aisément réhydratables, même après une période prolongée de conservation.

Les conditions générales du procédé de préparation des gels au moins partiellement déshydratés, incluant les micro-organismes, sont les suivantes :

a) on réalise tout d'abord une culture des micro-organismes dans un milieu de culture approprié contenant une source de carbone, en particulier des hydrates de carbone, jusqu'à obtention de la phase stationnaire, ce qui correspond en particulier pour les levures à un faible taux de bourgeonnement ou de division cellulaire, qui est de préférence inférieur à 5%.

b) On réalise une inclusion des micro-organismes présents dans le milieu de culture dans un polymère aisément gélifiable ou solidifiable, comme cela est classique.

Cette inclusion peut être faite par la technique classique de formation de gouttelettes de manière à obtenir des billes de gel incluant les micro-organismes.

De préférence, selon l'invention, on réalise une inclusion à double couche de manière à obtenir une couche extérieure ou enveloppe protectrice de gel sensiblement exempt de cellules de micro-organismes.

c) Le gel ainsi formé, notamment sous forme de billes ou de fibres, est trempé dans une solution contenant une forte concentration en substances hydrophiles, telle que précédemment définie, constituée de préférence par un sucre.

Dans le cas de l'emploi de saccharose, la concentration en substances hydrophiles est de préférence au

4

moins égale à 500 g/l, encore mieux environ 1000 g/l de solution de polymère.

Ce trempage est réalisé jusqu'à obtention d'un équilibre entre la solution et le gel.

d) On procède ensuite au séchage de ce gel ainsi trempé, après séparation de celui-ci de la solution contenant les substances hydrophiles précitées en utilisant tout traitement de séchage approprié antérieurement connu.

On peut utiliser par exemple la technique du lit fluidisé, la lyophilisation ou encore une enceinte de déshydratation contenant un sel de déshydratation.

e) On peut ensuite conserver le gel incluant un microorganisme, au moins partiellement déshydraté, sous atmosphère enrichie en $CO_2$.

f) Les particules peuvent ensuite être réhydratées selon la méthodologie habituellement utilisée pour les levures sèches déjà commercialisées, en vue de leur utilisation.

D'autres buts, caractéristiques et avantages de l'invention apparaîtront clairement à la lumière de la description explicative qui va suivre faite en référence aux exemples de réalisation ci-après en corrélation avec les figures annexées. Ces exemples sont naturellement donnés simplement à titre d'illustration de l'invention et ne sauraient donc en aucune façon être interprétés comme constituant une limitation de la portée de l'invention. Dans la présente description et les exemples, tous les pourcentages sont donnés en poids, sauf indication contraire.

Exemple I : Préparation de gel selon l'invention au moins partiellement déshydraté en utilisant comme micro-organisme une souche de Saccharomyces cerevisiae

Dans cet exemple, on utilise comme souche de microorganismes, une souche de Saccharomyces cerevisiae.

On utilise comme milieu de culture un Malt Wickerham à 10 g/l de glucose comme source hydrocarbonée.

On cultive les levures en erlenmeyer sur une table d'agitation à 28°C et on effectue une récolte après 32 h de culture à un stade où les micro-organismes ont un faible taux de bourgeonnement, n'excédant pas de préférence 5%, et une teneur en substances de réserve glycogène et tréhalose ici respectivement de 15 et 8% par dosage d'après les techniques décrites par HERBERT et al. dans Methods in Microbiology, Volume 5B, 1971, pages 210 à 344.

On peut obtenir selon une variante, dans les mêmes conditions de culture, des taux en substances de réserve supérieurs, par exemple glycogène environ 22% et tréhalose environ 13% en utilisant du Malt Wickerham modifié.

On centrifuge le milieu de culture de manière à séparer les micro-organismes que l'on met ensuite en suspension dans une solution aqueuse. Celle-ci est mélangée ensuite dans une solution aqueuse d'alginate de sodium "CECA SG 1100" de manière à obtenir une solution finale à 1,5%.

Le mélange est ensuite pompé et goutté dans une solution de $CaCl_2$ 0,2M à pH = 7.

Après 45 min de contact, les particules durcies, se présentant sous forme de billes d'alginate de calcium incluant les cellules de micro-organismes, sont rincées à l'eau distillée et présentent un diamètre moyen de 2,7 mm. Ces billes contiennent environ $5.10^8$ cellules de micro-organismes/ml de billes si ces préparations sont destinées à une prise de mousse selon la technique champenoise, ou $2.10^8$ cellules/ml de billes pour des procédés de fermentation alcoolique (réaction en cuves closes ou batches d'obtention de boissons fermentées ou de production d'éthanol).

Selon une caractéristique essentielle du procédé selon l'invention, après formation de ces billes, on trempe ces billes incluant les micro-organismes dans une solution contenant une forte concentration en substances hydrophiles de faible poids moléculaire, ici constituée par une solution de saccharose ayant une concentration de 100 g/100 ml. Cette immersion des billes dans cette solution de saccharose est maintenue jusqu'à obtention d'un équilibre de manière à obtenir une concentration finale dans les billes de saccharose égale à environ 100 g/100 ml de gel.

On peut également utiliser, au lieu du saccharose, d'autres sucres tels que glucose ou le fructose. On peut également utiliser des substances hydrophiles présentant une structure vitreuse après déshydratation comme le sorbitol, le glycérol, l'inositol.

Les particules ainsi imprégnées sont égouttées puis introduites dans un sécheur à lit fluidisé par exemple type RETSCH disponible chez Bioblock Scientifics, présenté au catalogue 1988 en page 152, à flux d'air et températures programmables entre 900-1800 l/min et 40-120°C respectivement.

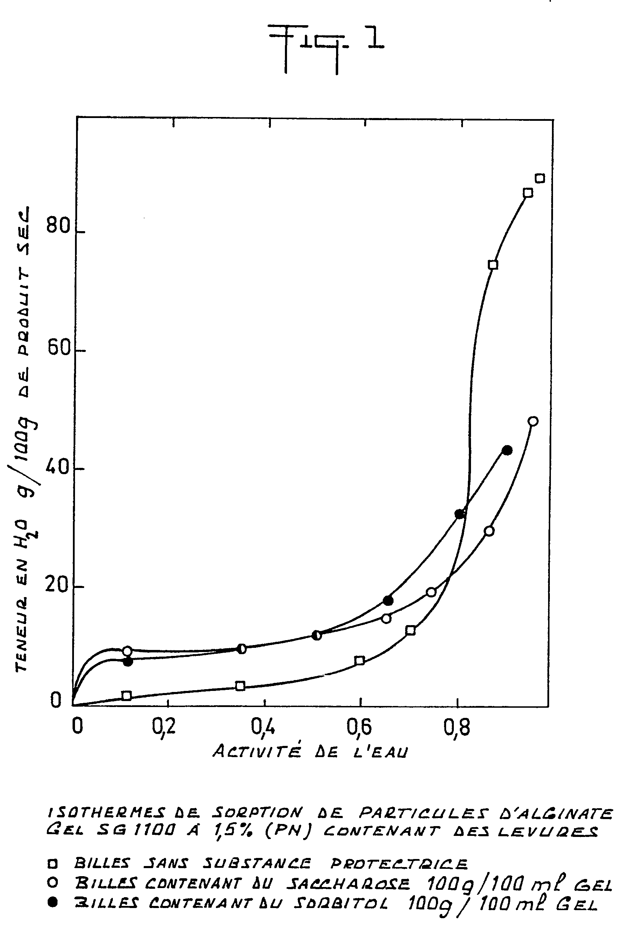

Les isothermes de désorption des billes sans les substances hydrophiles protectrices selon l'invention, et contenant en tant que substances hydrophiles protectrices selon l'invention du saccharose et du sorbitol à la concentration de 100 g/100 ml sont rapportées à la figure 1.

L'équilibre des billes est obtenu par désorption sous différentes atmosphères contrôlées par des solutions saturées à 25°C.

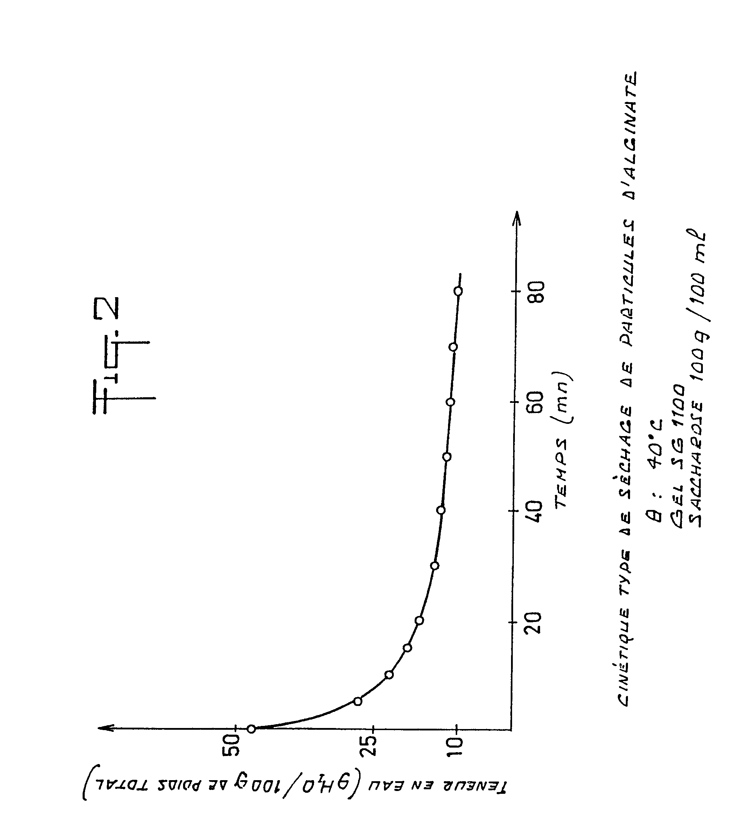

A la figure 2, on a tracé la courbe de séchage réalisée à 40°C en utilisant de l'air ambiant (humidité relative voisine de 65%).

Selon une caractéristique avantageuse, on atteint une activité de l'eau $a_w$ égale à environ 0,2 en utilisant de l'air plus sec ou encore une température de séchage plus élevée. L'expérience a montré que les cellules de micro-organismes conservaient leur viabilité si en fin de déshydratation on augmentait la température à environ 50°C.

On a rapporté au tableau 1 ci-après les viabilités observées lors de deux traitements de déshydratation par lit fluidisé jusqu'à obtention d'une activité de l'eau comprise entre 0,3 et 0,4.

### Tableau 1 : % de viabilités observées au cours de deux traitements de séchage à 40°C en fonction de l'état de la culture

| Stade de la culture | Récolte | Avant séchage | Après séchage |
|---|---|---|---|
| Début de phase stationnaire 25 % bourgeons | 99 | 99 | < 25 |
| 32 h de culture 3 % bourgeons | 99 | 99 | > 90 |

Les mesures de viabilité ont été effectuées après réhydratation des billes ou particules dans une solution de saccharose à 24 g/l (qui est proche de la composition du vin) pendant 1 h à la température ambiante (25°C) en milieu agité.

Les billes qui étaient translucides au départ redeviennent opaques après la réhydratation.

La teneur en eau finale est identique après réhydratation à la teneur en eau initiale.

On réalise une redissolution des billes par l'emploi d'une solution stérile de citrate de sodium glucosée ayant la composition suivante :

| | | |
|---|---|---|
| Peptone pancréatique | 1,2 | g |
| Glucose | 10 | g |
| NaCl | 10 | g |
| Trisodium citrate | 20 | g |
| Eau distillée q.s.p | 1 000 | ml |

On observe que les levures obtenues présentent une remarquable stabilité en pots scellés à température ambiante puisque après 15 jours de conservation, on obtient 86% de viabilité et après 2 mois, la viabilité reste supérieure à 50%.

Il est à noter en outre qu'un abaissement de la température de stockage à 4°C permet de maintenir une viabilité supérieure à 90% après la même période de stockage.

Egalement, l'utilisation dans une atmosphère de conservation essentiellement à base de $CO_2$, à température ambiante, améliore la survie puisqu'on obtient après 17 jours de conservation, des micro-organismes, ici des levures, présentant 94% d'activité.

Des reprises en vin de base dans le procédé sont tout à fait satisfaisantes.

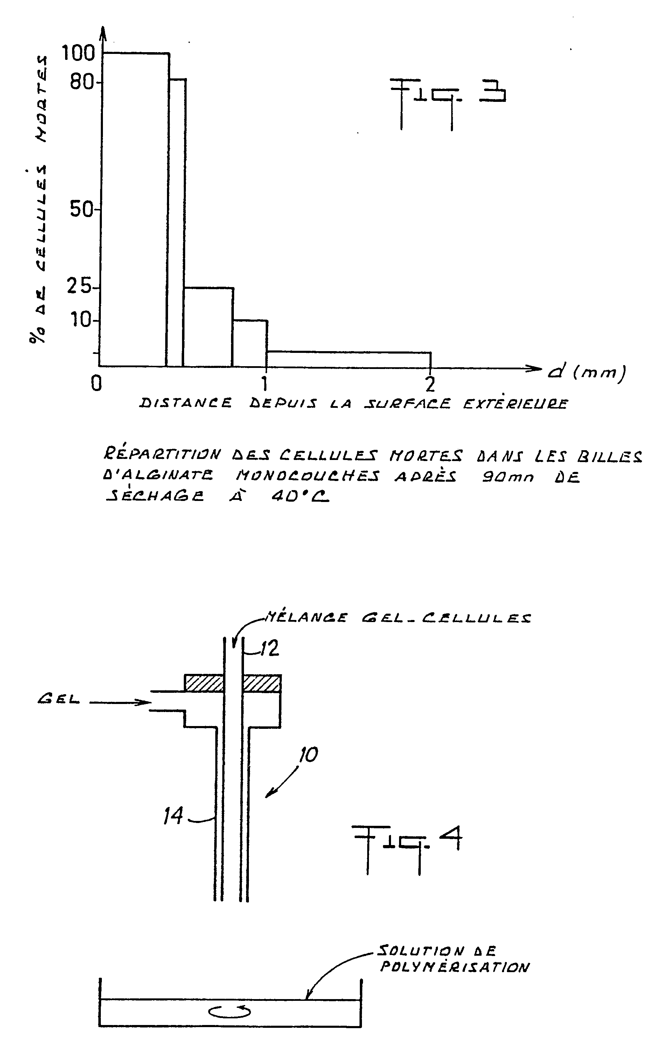

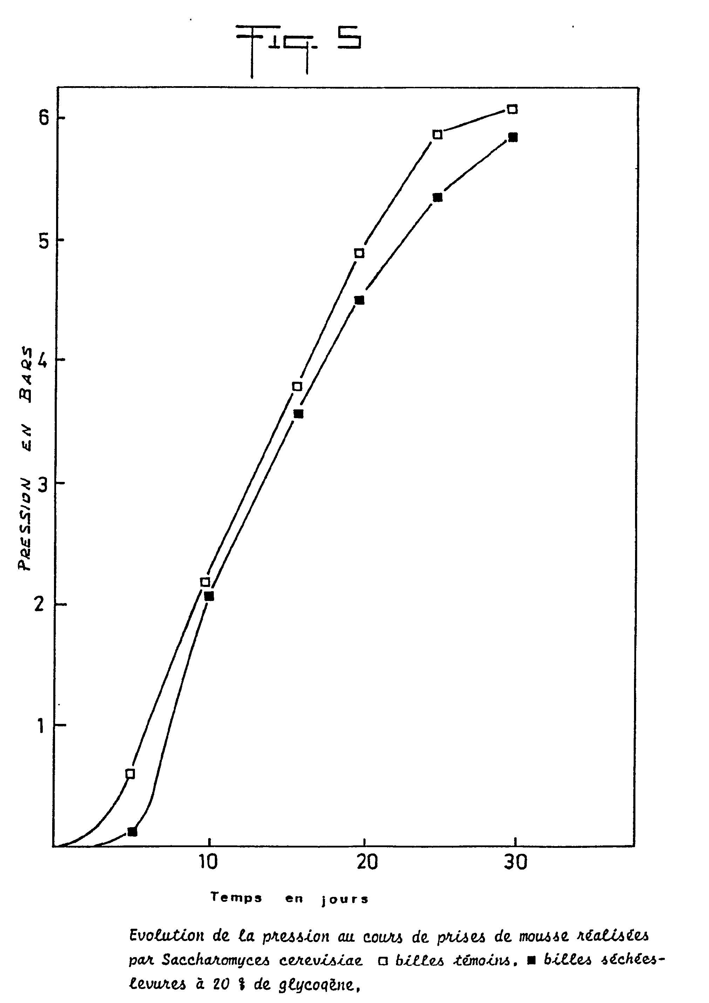

Une courbe d'évolution de la pression réalisée à l'aide de billes séchées ou déshydratées selon l'invention inclut des levures qui ont été cultivées jusqu'à obtention d'un taux de glycogène d'environ 20% conformément à cet exemple, en comparaison avec des billes témoins préparées avec les mêmes levures et non soumises à un séchage, fait l'objet de la figure 5.

On peut constater à partir de la courbe d'évolution des pressions au cours de prise de mousses que les billes obtenues selon l'invention après séchage conformément au procédé selon l'invention et réhydratation ont un comportement essentiellement similaire aux billes témoins non soumises à un tel séchage.

## Exemple II : Elaboration de gel double couche

On a pu observer que la préparation des micro-organismes inclus, par exemple dans des billes, selon l'exemple I, aboutit à une très bonne survie.

Cependant, des recherches complémentaires ont permis de déterminer que les micro-organismes morts n'étaient pas répartis uniformément dans la sphère du gel, de préférence de l'alginate.

La technique de dissolution précédemment décrite permet de mettre en évidence cette répartition.

On a représenté à la figure 3 le schéma de la répartition spatiale des cellules mortes dans les particules de l'exemple I, qui sont monocouches, depuis la surface extérieure des particules.

91% des cellules mortes se répartissent dans les 400 premiers microns d'une sphère de 4 mm de diamètre.

Les expériences réalisées par les inventeurs ont permis de démontrer que l'on peut améliorer sensiblement la survie des micro-organismes si l'on prépare des gels double couche, c'est-à-dire comprenant une couche interne ou noyau incluant les cellules de micro-organismes et une couche externe ou enveloppe de protection sensiblement dépourvue de cellules de micro-organismes. La technique correspondante pour la préparation de gel double couche, en particulier sous forme de billes, est connue dans la littérature présentée en début de description, en particulier à partir de GB-1158662 ou le document Microcapsules Processing and Technology (Asaji Kondo) 1979, page 62.

Pour une meilleure compréhension, le schéma de principe d'une buse à deux tubes concentriques est représenté à la figure 4 pour rappel. Cette buse 10 comprend un tube 12 axial, central dans lequel on introduit le mélange d'une solution de gel, par exemple un gel d'alginate de sodium contenant en suspension les cellules de micro-organismes.

Dans le tube externe 14 concentrique au tube interne 12, on introduit la solution de gel seule, exempte de cellules de micro-organismes, par exemple de l'alginate de sodium, de manière à former des gouttes double couche comme cela est connu.

Il est préféré selon l'invention que la couche externe formant enveloppe protectrice présente une épaisseur de 0,35 à 0,70 mm environ pour un diamètre des billes ou gouttelettes d'environ 4 mm.

Grâce à cette structure double couche, on aboutit donc à éviter de tuer des cellules de micro-organismes lors du séchage réalisé comme précédemment décrit.

## Exemple III : Préparation de gel incluant du Saccharomyces uvarum

On procède comme indiqué à l'exemple I, si ce n'est que l'on utilise comme souche de micro-organismes une souche de brasserie, Saccharomyces uvarum.

On obtient les mêmes résultats avec une viabilité supérieure à 90%.

Les reprises en moûts de bière de 12,5 Plato et 80% de sucres fermentes cibles sont tout à fait satisfaisantes.

## Exemple IV : Préparation de gel incluant du Schizosaccharomyces

On procède comme indiqué à l'exemple I, si ce n'est que l'on utilise comme micro-organismes une souche classique de Schizosaccharomyces. Cette souche est réputée pour donner de très mauvaises survies dans des conditions de déshydratation classique.

Cependant, selon l'invention, de manière totalement inattendue, on obtient des résultats comparables aux autres souches avec une viabilité supérieure à 90% après séchage et réhydratation.

## Exemple V : Préparation de gel incluant des cellules de Saccharomyces cerevisiae par déshydratation sous atmosphère contrôlée

On prépare des billes comme à l'exemple I.

Les billes sont placées dans des pots twist-off étanches (750 ml) dont l'humidité de l'air est contrôlée par des solutions saturées de sels ou des solutions de glycérol.

L'activité de l'eau est vérifiée par réfractométrie pour les solutions de glycérol ou par l'appareil NOVASINA EJ 3 pour les solution de sels.

L'équilibre des billes se fait par désorption. Après 250 à 300 h de mise en équilibre, la viabilité est mesurée comme précédemment décrit.

Les résultats obtenus sont en général inférieurs à ceux obtenus par un séchage plus rapide en lit fluidisé.

On obtient les meilleurs taux de conservation avec une activité de l'eau inférieure à 0,5.

A une activité de 0,8 ou davantage, la survie des micro-organismes est inférieure à 5% tandis qu'à une activité de 0,11 elle est supérieure à 40%.

Exemple VI : Préparation de gel incluant des cellules de Saccharomyces cerevisiae, par lyophilisation

La préparation des billes est identique à l'exemple I en utilisant de préférence le saccharose comme substance hydrophile protectrice à la même concentration.

Dans les conditions opératoires, une température de congélation de −30°C entraîne une moins bonne conservation des structures des particules qu'une température de −75 à −80°C avec une vitesse de refroidissement plus rapide.

Les particules congelées sont disposées dans un lyophilisateur USIFROID SMJ.

La sublimation est pratiquée sous vide poussé (< 0,025 mmHg), le réchauffement est effectué par paliers à −40°C, 0°C et 20°C.

Le cycle complet dure 24 h pour aboutir à une activité de l'eau de 0,4.

Les taux de survie des cellules de micro-organismes obtenues sont toujours supérieurs à 80°C.

Les conditions de réhydratation sont identiques à celles utilisées dans l'exemple I.

## Revendications

1. Procédé de préparation de micro-organismes inclus dans des gels au moins partiellement déshydratés présentant une viabilité améliorée après réhydratation, comprenant :
   a) l'inclusion de micro-organismes dans une solution de polymère susceptible de se transformer en gel ;
   b) la gélification de cette solution contenant les micro-organismes de manière à former un gel incluant les micro-organismes ; et
   c) le séchage de ce gel incluant les micro-organismes, pour obtenir un gel au moins partiellement déshydraté,
   caractérisé en ce qu'on prépare des gels incluant les micro-organismes ayant une forte concentration en substances hydrophiles que l'on soumet ensuite à ladite étape de séchage.

2. Procédé selon la revendication 1, caractérisé en ce que la substance hydrophile est d'un faible poids moléculaire.

3. Procédé selon la revendication 1 ou 2, caractérisé en ce que la substance hydrophile précitée est choisie parmi les polyols, tels que le sorbitol, l'inositol, le glycérol ; les sucres, tels que le saccharose, le glucose, le fructose ; et encore mieux le saccharose.

4. Procédé selon l'une des revendications 1 à 3, caractérisé en ce qu'on utilise le saccharose en une concentration au moins égale à 500 g/l, de préférence environ 1000 g/l de solution de polymère susceptible de se transformer en gel.

5. Procédé selon l'une des revendications 1 à 4, caractérisé en ce qu'on réalise une culture des cellules de micro-organismes jusqu'à obtention de la phase stationnaire, ce qui correspond en particulier pour les levures à un faible taux de bourgeonnement ou d'initiation de division, de préférence inférieur à 5%.

6. Procédé selon l'une des revendications 1 à 5, caractérisé en ce qu'on prépare un gel ayant une structure double couche comprenant une couche interne ou noyau de gel contenant les cellules de micro-organismes et une couche externe ou enveloppe de gel sensiblement exempt de micro-organismes.

7. Procédé selon la revendication 6, caractérisé en ce que l'épaisseur de la couche externe ou enveloppe, dans le cas de billes ayant un diamètre d'environ 4 mm, est inférieure à 0,8 mm.

8. Procédé selon l'une des revendications 1 à 7, caractérisé en ce qu'on obtient la forte concentration en substances hydrophiles précitée dans le gel par trempage du gel dans une solution de substances hydrophiles ayant ladite forte concentration, jusqu'à l'équilibre, puis en soumettant le gel à l'étape de séchage précitée.

9. Procédé selon l'une des revendications 1 à 8, caractérisé en ce qu'on réalise un séchage du gel incluant les micro-organismes, ayant la forte concentration précitée en substances hydrophiles, jusqu'à obtention d'une activité de l'eau inférieure à environ 0,5.

10. Procédé selon l'une des revendications 1 à 9, caractérisé en ce qu'on réalise le séchage précité à une température voisine de 40°C et de préférence on augmente la température jusqu'à atteindre environ 50°C en fin de séchage.

11. Procédé selon l'une des revendications 1 à 10, caractérisé en ce qu'on réalise le séchage précité du gel incluant les micro-organismes, comprenant une forte concentration en substances hydrophiles, dans un lit fluidisé.

12. Procédé selon l'une des revendications 1 à 10, caractérisé en ce qu'on réalise un séchage du gel contenant les micro-organismes, comprenant la forte concentration précitée en substances hydrophiles par une technique de lyophilisation sous vide, de préférence à une température de lyophilisation de l'ordre de −80°C ± 10°C environ.

13. Procédé selon l'une des revendications 1 à 12, caractérisé en ce qu'on conserve le gel contenant les micro-organismes, comprenant la forte concentration en substances hydrophiles précitée, au moins partiellement déshydratées, dans un emballage étanche à la vapeur d'eau, qui est de préférence maintenu à une température relativement basse, de préférence d'environ 4°C.

14. Procédé selon la revendication 13, caractérisé en ce que cette conservation a lieu dans une atmosphère contrôlée très appauvrie en oxygène et enrichie en $CO_2$.

15. Procédé selon l'une des revendications 1 à 14, caractérisé en ce que les micro-organismes sont des levures, en particulier du genre Saccharomyces, Schizosaccharomyces.

16. Utilisation des gels incluant des micro-organismes, au moins partiellement déshydratés contenant une forte proportion en substances hydrophiles pour la préparation de boissons fermentées ou refermentées, ainsi que pour la préparation d'alcool éthylique, en particulier ceux obtenus par le procédé selon l'une quelconque des revendications 1 à 15.

17. Gels incluant des micro-organismes ayant une forte concentration en substances hydrophiles.

18. Gels selon la revendication 17, caractérisés en ce que ces gels sont obtenus par le procédé selon l'une quelconque des revendications 1 à 15, éventuellement à un état réhydraté.


## Patentansprüche

1. Verfahren zur Herstellung von in zumindest teilweise dehydratisierten und nach der Rehydration eine verbesserte Lebensfähigkeit aufweisenden Gelen eingeschlossenen Mikroorganismen, umfassend :

a) den Einschluß von Mikroorganismen in eine in ein Gel überführbare Polymerlösung ;

b) die Gelatinierung dieser die Mikroorganismen enthaltenden Lösung zur Bildung eines die Mikroorganismen einschließenden Gels ; und

c) die Trocknung dieses die Mikroorganismen einschließenden Gels zur Gewinnung eines zumindest teilweise dehydratisierten Gels ; dadurch gekennzeichnet,

daß man die Mikroorganismen einschließende Gele mit einer hohen Konzentration an hydrophilen Substanzen herstellt, welche man danach dem Trocknungsschritt unterzieht.

2. Verfahren nach Anspruch 1, dadurch gekennzeichnet, daß die hydrophile Substanz ein geringes Molekulargewicht aufweist.

3. Verfahren nach Anspruch 1 oder 2, dadurch gekennzeichnet, daß die hydrophile Substanz ausgewählt ist aus Polyolen, wie Sorbit, Inosit, Glyzerin ; Zuckern, wie Saccharose, Glucose, Fructose ; und noch besser Saccharose.

4. Verfahren nach einem der Ansprüche 1 bis 3, dadurch gekennzeichnet, daß die Saccharose in einer Konzentration von mindestens 500 g/l, vorzugsweise etwa 1000 g/l an in Gel überführbarer Polymerlösung eingesetzt wird.

5. Verfahren nach einem der Ansprüche 1 bis 4, dadurch gekennzeichnet, daß eine Kultur aus Zellen von Mikroorganismen bis zur Erlangung der stationären Phase hergestellt wird, was insbesondere bei den Hefen mit einem geringen Grad an Knospenbildung oder Teilungsansatz, vorzugsweise unter 5%, übereinstimmt.

6. Verfahren nach einem der Ansprüche 1 bis 5, dadurch gekennzeichnet, daß ein Gel mit einer Doppelschichtstruktur hergestellt wird, umfassend eine Innenschicht oder einen Kern aus Gel, welche die Mikroorganismuszellen enthält, und eine Außenschicht oder Hülle aus Gel, die im wesentlichen frei von Mikroorganismen ist.

7. Verfahren nach Anspruch 6, dadurch gekennzeichnet, daß die Dicke der Außenschicht oder Hülle bei Kugeln mit einem Durchmesser von etwa 4 mm weniger als 0,8 mm beträgt.

8. Verfahren nach einem der Ansprüche 1 bis 7, dadurch gekennzeichnet, daß die hohe Konzentration an hydrophilen Substanzen im Gel durch Tauchen des Gels in eine Lösung von hydrophilen Substanzen mit der besagten hohen Konzentration bis zum Gleichgewicht und anschließendes Unterziehen des Gels der Trocknungsstufe erzielt wird.

9. Verfahren nach einem der Ansprüche 1 bis 8, dadurch gekennzeichnet, daß eine Trocknung des die Mikroorganismen einschließenden Gels mit der hohen Konzentration an hydrophilen Substanzen bis zur Erzielung einer Wasseraktivität von weniger als etwa 0,5 durchgeführt wird.

10. Verfahren nach einem der Ansprüche 1 bis 9, dadurch gekennzeichnet, daß die Trocknung bei einer Temperatur nahe 40°C durchgeführt und die Temperatur vorzugsweise erhöht wird, bis sie am Ende der Trocknung etwa 50°C erreicht.

11. Verfahren nach einem der Ansprüche 1 bis 10, dadurch gekennzeichnet, daß die Trocknung des die Mikroorganismen einschließenden Gels mit einer hohen Konzentration an hydrophilen Substanzen in einem Fließbett durchgeführt wird.

12. Verfahren nach einem der Ansprüche 1 bis 10, dadurch gekennzeichnet, daß eine Trocknung des die Mikroorganismen enthaltenden Gels mit der hohen Konzentration an hydrophilen Substanzen mittels einer Lyophilisationsmethode unter Vakuum, vorzugsweise bei einer Lyophilisationstemperatur im Bereich von etwa –80°C ± 10°C, durchgeführt wird.

13. Verfahren nach einem der Ansprüche 1 bis 12, dadurch gekennzeichnet, daß das die Mikroorganismen enthaltende Gel mit der hohen Konzentration an hydrophilen Substanzen, die zumindest teilweise dehydratisiert sind, in einer wasserdampfdichten Verpackung, die vorzugsweise auf einer relativ niedrigen Temperatur, vorzugsweise etwa 4°C, gehalten wird, konserviert wird.

14. Verfahren nach Anspruch 13, dadurch gekennzeichnet, daß die Konservierung in einer kontrollierten Atmosphäre, die sehr arm an Sauerstoff und mit $CO_2$ angereichert ist, stattfindet.

15. Verfahren nach einem der Ansprüche 1 bis 14, dadurch gekennzeichnet, daß die Mikroorganismen Hefen, insbesondere der Gattung Saccharomyces, Schizosaccharomyces, sind.

16. Verwendung der zumindest teilweise dehydratisierten und einen hohen Anteil an hydrophilen Substanzen aufweisenden Gele, welche Mikroorganismen einschließen, insbesondere die mit dem Verfahren nach einem der Ansprüche 1 bis 15 erhaltenen, zur Herstellung von vergorenen oder wiedervergorenen Getränken sowie zur Herstellung von Ethylalkohol.

17. Mikroorganismen einschließende Gele mit einer hohen Konzentration an hydrophilen Substanzen.

18. Gele nach Anspruch 17, dadurch gekennzeichnet, daß die Gele mittels des Verfahrens nach einem der Ansprüche 1 bis 15, gegebenenfalls in einem rehydrierten Zustand, erhalten sind.

## Claims

1. Process for the preparation of micro-organisms enclosed in at least partially dehydrated gels exhibiting an improved viability after rehydration, comprising :

a) the inclusion of micro-organisms in a polymer solution capable of being transformed into a gel ;

b) the gelation of this solution containing the micro-organisms so as to form a gel enclosing the micro-organisms ; and

c) the drying of this gel enclosing the micro-organisms in order to obtain an at least partially dehydrated gel,

characterized in that gels enclosing the micro-organism having a high concentration of hydrophilic substances are prepared which are then subjected to the said drying step.

2. Process according to Claim 1, characterized in that the hydrophilic substance is one of low molecular weight.

3. Process according to Claim 1 or 2, characterized in that the above-mentioned hydrophilic substance is selected from the polyols, such as sorbitol, inositol, glycerol ; the sugars, such as sucrose, glucose, fructose, and preferably sucrose.

4. Process according to one of the Claims 1 to 3, characterized in that sucrose is used at a concentration at least equal to 500 g/l, and preferably about 1000 g/l of polymer solution capable of being transformed into a gel.

5. Process according to one of the Claims 1 to 4, characterized in that a culture of cells of micro-organisms is grown until the stationary phase is attained, which corresponds in particular in the case of the yeasts to a low degree of budding or initiation of division, preferably less than 5%.

6. Process according to one of the Claims 1 to 5, characterized in that a gel having a double layer structure is prepared comprising an internal layer or core of gel containing the cells of micro-organisms and an external layer or envelope of gel practically devoid of micro-organisms.

7. Process according to Claim 6, characterized in that the thickness of the external layer or envelope, in the case of beads having a diameter of about 4 mm, is less than 0.8 mm.

8. Process according to one of the Claims 1 to 7, characterized in that the above-mentioned high concentration of hydrophilic substances in the gel is obtained by soaking the gel in a solution of hydrophilic substances having the said high concentration, up to equilibrium, then by subjecting the gel to the above-mentioned drying step.

9. Process according to one of the Claims 1 to 8, characterized in that a drying of the gel enclosing the micro-organisms, having the above-mentioned high concentration of hydrophilic substances, is carried out until an activity of water lower than about 0.5 is obtained.

10. Process according to one of the Claims 1 to 9, characterized in that the above-mentioned drying is performed at a temperature close to 40°C and the temperature is preferably increased until about 50°C is attained at the end of drying.

11. Process according to one of the Claims 1 to 10, characterized in that the above-mentioned drying of the gel enclosing the micro-organisms and containing a high concentration of hydrophilic substances is performed in a fluidized bed.

12. Process according to one of the Claims 1 to 10, characterized in that a drying of the gel containing the micro-organisms, and containing the above-mentioned high concentration of hydrophilic substances, is performed by means of a technique of lyophilization in a vacuum, preferably at a temperature of lyophilization of the order of about −80°C ± 10°C.

13. Process according to one of the Claims 1 to 12, characterized in that the gel containing the micro-organisms, and containing the above-mentioned high concentration of at least partially dehydrated hydrophilic substances, is stored in a water vapour-tight packing which is preferably maintained at a relatively low temperature, preferably about 4°C.

14. Process according to Claim 13, characterized in that this preservation takes place in a controlled atmosphere very poor in oxygen and enriched in $CO_2$.

15. Process according to one of the Claims 1 to 14, characterized in that the micro-organisms are yeasts, in particular of the genus Saccharomyces and Schizosaccharomyces.

16. Use of the gels enclosing at least partially dehydrated micro-organisms containing a high concentration of hydrophilic substances for the preparation of fermented or refermented drinks as well as for the preparation of ethyl alcohol, in particular those obtained by the process according to one of the Claims 1 to 15.

17. Gels containing micro-organisms containing a high concentration of hydrophilic substances.

18. Gels according to Claim 17, characterized in that said gels are obtained by the process according to one of the Claims 1 to 15, optionally in a rehydrated condition.

Fig. 1

ISOTHERMES DE SORPTION DE PARTICULES D'ALGINATE
GEL SG 1100 À 1,5% (PN) CONTENANT DES LEVURES

□ BILLES SANS SUBSTANCE PROTECTRICE
O BILLES CONTENANT DU SACCHAROSE 100g/100 mℓ GEL
● BILLES CONTENANT DU SORBITOL 100g/100 mℓ GEL

Fig.2

CINÉTIQUE TYPE DE SÈCHAGE DE PARTICULES D'ALGINATE

θ : 40°c
GEL SG 1100
SACCHAROSE 100g / 100 ml

TEMPS (mn)

TENEUR EN EAU (g H₂O / 100 g DE POIDS TOTAL)

Fig. 3

RÉPARTITION DES CELLULES MORTES DANS LES BILLES D'ALGINATE MONOCOUCHES APRÈS 90mn DE SÉCHAGE À 40°C

Fig. 4

14

# Fig. 5

Evolution de la pression au cours de prises de mousse réalisées par Saccharomyces cerevisiae □ billes témoins, ■ billes séchées-levures à 20 % de glycogène,